# EUROPÄISCHE PATENTANMELDUNG

(11) **EP 3 470 122 A1**
(43) Veröffentlichungstag der Anmeldung: **17.04.2019**
(21) Anmeldenummer: 18000806.2
(22) Anmeldetag: 11.10.2018
(51) Int. Cl.: A63B 71/12, A41B 11/00, A61F 5/01

(54) **SPORTSTRUMPF ZUM SCHUTZ VOR VERLETZUNGEN**

(30) Priorität: 11.10.2017 DE 102017123608
(71) Anmelder: Allinnovation UG, 97500 Ebelsbach (DE)
(72) Erfinder: Dorsch, Benjamin, 97500 Ebelsbach (DE)
(74) Vertreter: Gleim, Christian Ragnar

(57) **Zusammenfassung**

Sportstrumpf 1 zum Schutz vor Verletzungen eines Spielers 10, welcher Sportstrumpf 1 ein elastisches Textil 11, 12 mit einem Fußabschnitt 11 und einem Beinabschnitt 12 umfasst, und wobei an dem Sportstrumpf 1
- mindestens ein erstes Dämpfungsteil 21, das am Beinabschnitt 12 derart angeordnet ist, um das Schienbein eines den Sportstrumpf 1 tragenden Spielers 10 abzudecken,
- mindestens ein zweites Dämpfungsteil 22, das am Beinabschnitt 12 derart angeordnet ist, um die Wade eines den Sportstrumpf 1 tragenden Spielers 10 abzudecken, und
- mindestens ein drittes Dämpfungsteil 23, das am Übergang zwischen Beinabschnitt 12 und Fußabschnitt 11 derart angeordnet ist, um einen Knöchel eines den Sportstrumpf 1 tragenden Spielers 10 abzudecken.

## Beschreibung

Die Erfindung betrifft einen Sportstrumpf zum Schutz vor Verletzungen eines Spielers gemäß dem unabhängigen Anspruch.

Es ist aus dem Stand der Technik bekannt, Sportstrümpfe speziell zum Schutz vor Verletzungen durch Fremdeinwirkungen in Form von Schlägen auf den Unterschenkel auszubilden. Ein besonderes Einsatzgebiet sind Sportstrümpfe zum Schutz vor Verletzungen für den Fußball-, Hockey- und Rugbysport, die insbesondere vor Verletzungen eines Spielers durch einen Gegenspieler schützen. Dabei fangen diese leichte und schwere Stöße während eines Spielverlaufs ab.

Aus der DE 199 25 081 A1 sind zum Beispiel Sportstrümpfe bekannt, die Schutz gegen Verletzungen am Schienbein ermöglichen, indem diese Stoßdämpfungselemente zum Abfedern von Tritten und Schlägen gegnerischer Spieler vorsehen. Derartige Stoßdämpfungselemente können zum Beispiel durch Luftkammern gebildet werden, die die Stoßenergie absorbieren. Die Luftkammern sind in einem flexiblen Strumpfmaterial eingebettet, indem diese in vom Strumpfmaterial gebildeten Taschen eingeschlossen sind.

Luftpolster in einem Schienbeinschoner vorzusehen ist auch aus der WO 99/ 00 161 bekannt, wobei die Luftpolster in einem separaten Stoßenergie absorbierenden Polsterelement angeordnet sind. Als Dämpfungsmaterial zur Absorption der Stoßenergie ist es weit verbreitet synthetische Gummimaterialen einzusetzen, die zum Beispiel aus der JP 2001 070 495 bekannt sind. Darin wird ein thermoplastischer Formgebungsprozess zu deren Herstellung beschrieben.

Es ist aus dem technischen Gebiet auch bekannt, viskose Kunststoffzusammensetzungen zur Herstellung von Schienbeinschonern einzusetzen. Die Absorption von Schlägen wird hier durch das Viskosematerial selbst bewirkt, so dass auf zusätzliche dämpfende Elemente verzichtet werden kann.

Eine besondere Form von Sportschonern zielt auf den Schutz der Wade. Derartige Wadenschoner sind bekannt als röhrenförmige elastische Abschnitte, die oberhalb des Knöchels an der Wade eng anliegend in der Art einer Wadenbandage oder eines Wadenstrumpfes getragen werden. Dazu werden Schutzpolster mittels Trägerbändern an der Wade angeordnet.

Es ist ebenfalls bekannt, Verletzungen der Knöchel mit weiteren Schutzelementen zu schützen, die separat angezogen werden. Es gibt eigene Knöchelschoner, die aus einem runden Textilabschnitt mit zwei gegenüberliegenden runden Schutzpolstern gebildet sind. Damit der Knöchelschoner nicht nach oben rutscht, ist eine um die Ferse getragene Schlaufe vorgesehen.

Zudem sind aus dem Stand der Technik die DE 203 18 851 U1, DE 10 2012 209 814 A1, DE 27 43 741 A1, DE 20 2005 002 803 U1, DE 20 2012 100 179 U1 und die DE 20 2007 001 955 U1 bekannt.

Der Nachteil dieser bekannten Lösungen ist, dass diese nicht gleichzeitig bequem getragen werden können und Schutz für Schienbein, Knöchel sowie Unterschenkel bieten. Außerdem kommt es für den Fall, dass nur Schutz für entweder Schienbein, Knöchel oder Unterschenkel besteht, zu einem Verletzungsrisiko der jeweils anderen ungeschützten Körperteile.

Diese Aufgabe wird durch einen Sportstrumpf zum Schutz vor Verletzungen eines Spielers gemäß dem unabhängigen Anspruch gelöst. Vorteilhafte Aspekte bilden den Gegenstand der jeweiligen Unteransprüche.

Die Erfindung umfasst einen Sportstrumpf zum Schutz vor Verletzungen eines Spielers, welcher Sportstrumpf ein elastisches Textil mit einem Fußabschnitt und einem Beinabschnitt umfasst. An dem Sportstrumpf ist mindestens ein erstes Dämpfungsteil, das am Beinabschnitt derart angeordnet ist, um das Schienbein eines den Sportstrumpf tragenden Spielers abzudecken, mindestens ein zweites Dämpfungsteil, das am Beinabschnitt derart angeordnet ist, um die Wade eines den Sportstrumpf tragenden Spielers abzudecken, und mindestens ein drittes Dämpfungsteil, das am Übergang zwischen Beinabschnitt und Fußabschnitt derart angeordnet ist, um einen Knöchel eines den Sportstrumpf tragenden Spielers abzudecken, angebracht. Aufgrund der Kombination von erstem, zweitem und drittem Dämpfungsteil in einem Sportstrumpf kann dieser im Vergleich zu den einzelnen Teilen bequem getragen werden. Außerdem wird ein Schutz für Schienbein, Knöchel sowie Unterschenkel gleichzeitig gewährleistet und vermieden, dass eines dieser Körperteile bei einem Spieler insbesondere während des Fußball-, Hockey- und Rugbysports, ungeschützt ist, weshalb damit das Verletzungsrisiko verringert ist. Das elastische Textil ist bevorzugt aus Nylon (Polyamid), Kevlar oder Dyneema gewoben. Die Dämpfungsteile sind aus einem Schaum (z.B. thermoplastisches Polyurethan (TPU)), der vorteilhafterweise im Randbereich thermisch gehärtet ist.

Gemäß einem besonders bevorzugten technischen Aspekt umfasst das erste Dämpfungsteil, das zweite Dämpfungsteil und das dritte Dämpfungsteil eine Kunststoffschale. Die Kunststoffschale ist zwischen dem (den Sportstrumpf bildenden) elastischen Textil und einem weiteren (zusätzlich am Sportstrumpf angeordneten) elastischen Textilabschnitt angeordnet. Auf diese Art und Weise kann eine geschlossene Tasche gebildet werden, zum Beispiel indem der elastische Textilabschnitt mit dem elastischen Textil verschweißt wird.

Eine vorteilhafte, technische Variante dafür sieht vor, dass die Kunststoffschale auf einer Seite eine Polsterschicht, insbesondere eine Schaumstoffschicht, umfasst. Die Polsterschicht kann aus einer mit großer Stärke gewebten Textilschicht bestehen. So kann mit der Kunststoffschale ein hartschaliges Element bereitgestellt werden, das zum Körper des Spielers hin durch eine speziell am Sportstrumpf selbst vorgesehene Schicht abfedernd wirkt.

Ein weiterer besonders bevorzugter Aspekt sieht vor, dass das elastische Textil abschnittsweise aus einem transparenten Material ist. Dazu kann das elastische Material selbst transparent gefertigt werden oder ein transparenter Materialabschnitt eingefügt werden.

Ein anderer vorteilhafter Aspekt sieht vor, dass das dritte Dämpfungsteil einen ersten Dämpfungsteilabschnitt, der am Übergang zwischen Beinabschnitt und Fußabschnitt derart angeordnet ist, um eine erste Knöchelseite eines den Sportstrumpf tragenden Spielers abzudecken, und einen zweiten Dämpfungsteilabschnitt, der am Übergang zwischen Beinabschnitt und Fußabschnitt derart angeordnet ist, um eine zweite Knöchelseite eines den Sportstrumpf tragenden Spielers abzudecken, umfasst. Die beiden zweiten Dämpfungsteilabschnitte können miteinander derart verbunden sein, um den Fuß im Bereich des Knöchels von hinten zu umgeben, wobei die beiden Dämpfungsteilabschnitte dabei derart geformt sind, um sich nach vorne über die Seiten des Knöchels zu erstrecken.

Ein anderer besonders bevorzugter Aspekt sieht vor, dass das dritte Dämpfungsteil Befestigungsöffnungen zum Verschnüren mit einem Schuh des den Sportstrumpf tragenden Spielers umfasst. Das Verschnüren des Dämpfungsteils mit dem Sportschuh bewirkt eine Stützung des Knöchels, um speziell Bänderverletzungen vorzubeugen.

Besonders bevorzugt hat das erste Dämpfungsteil eine Breite (in Richtung des Umfangs des Unterschenkels des Spielers) im Bereich von 10 cm bis 20 cm und eine Höhe (in Richtung der Länge des Schienbeins des Spielers) im Bereich von 20 cm bis 30 cm. Diese Größe bezieht sich auf ein Rechteck oder Formen mit ungleichen, gegenüberliegenden Seitenlängen.

Ein anderer besonders bevorzugter Aspekt betrifft ein zweites Dämpfungsteil mit einer Breite im Bereich von 5 cm bis 15 cm und einer Höhe im Bereich von 20 cm bis 30 cm. Auch hier bezieht sich die Größe ebenfalls auf ein Rechteck oder auf Formen mit ungleichen, gegenüberliegenden Seitenlängen.

Gemäß einem anderen bevorzugten Aspekt umfasst der Sportstrumpf ferner hochelastische Stützbänder, die eine im Vergleich zum elastischen Textil höhere Elastizität aufweisen. Die elastischen Stützbänder sind einstückig mit dem Sportstrumpf als Abschnitte des elastischen Textils oder als separate Stützbänder ausgebildet.

Es ist außerdem bevorzugt, dass der Sportstrumpf ferner hochelastische Stützbänder umfasst, die eine im Vergleich zum elastischen Textil höhere Elastizität aufweisen. Die elastischen Stützbänder sind an dem Sportstrumpf als Abschnitte des elastischen Textils, nur mit höherer Elastizität, oder als separate (am elastischen Textil angeordnete) Elemente ausgebildet. Die hochelastischen Stützbänder haben eine komprimierende Wirkung, wodurch die Durchblutung erhöht wird. Gemäß einer vorteilhaften Variante kann eine gummiartige Schicht, insbesondere im Bereich der hochelastischen Stützbänder, angeordnet sein, um ein Verrutschen zu verhindern.

Gemäß einem besonders bevorzugten Aspekt ist die Elastizität der hochelastischen Stützbänder derart gewählt, dass die Rückstellkraft im Vergleich zu dem elastischen Textil um mehr als 10 % größer ist. Besonders bevorzugt ist ein Bereich von 50 % bis 100 % für die Elastizität.

Vorteilhafterweise umfassen die Stützbänder ein Wadenstützband, das die Wade vollständig entlang des Umfangs umgibt, ein Knöchelstützband, das den Fuß im Knöchelbereich vollständig entlang des Umfangs umgibt, ein Fersenstützband, das sich von der Sohle über die Ferse entlang der Wade erstreckt und ein Fußrückenstützband, das den Fuß im Bereich der Fußbrücke vollständig entlang des Umfangs umgibt.

Gemäß einem vorteilhaften Aspekt umfasst das elastische Textil zumindest eines der folgenden Materialien aus der Gruppe Nylon, Kevlar, Glasfasern, Dyneema, Polyester, Polyurethan, Thermoplastisches Polyurethan.

Vorteilhafterweise ist das elastische Textil mit einem Kunststoffharz getränkt ist, welches Kunststoffharz derart ausgebildet ist, um bei Kontakt mit Wasser zu erhärten. Das Kunststoffharz ist aus einem Material wie bei einem Cast (Kunststoffgips) aus der Medizin.

Eine weitere Variante sieht vor, dass die Kunststoffschale und das elastischen Textil einstückig ausgebildet ist.

Im Folgenden wird die Erfindung anhand der in den beigefügten Zeichnungen dargestellten Beispiele näher erläutert. Es zeigen:
- Fig. 1: ein Ausführungsbeispiel für einen Sportstrumpf zum Schutz vor Verletzungen eines Spielers mit drei Dämpfungsteilen;
- Fig. 2: die Anordnung von Stützbändern in einem Sportstrumpf gemäß Fig. 1; und
- Fig. 3: ein Beispiel für den Aufbau eines Dämpfungsteils für einen Sportsstrumpf aus Fig. 1.

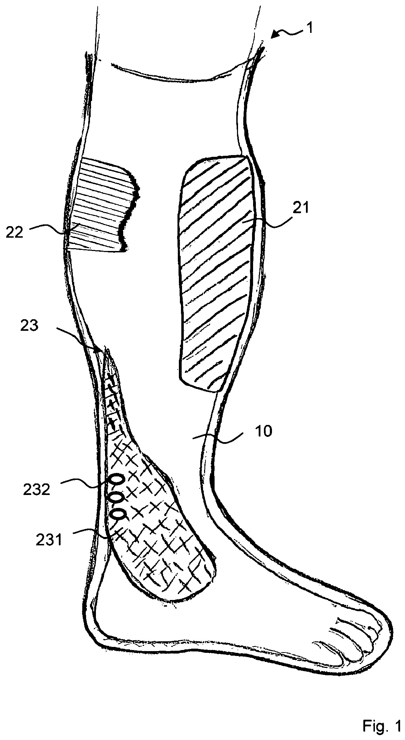

In **Fig. 1** ist ein Beispiel für einen Sportstrumpf 1 zum Schutz vor Verletzungen eines Spielers 10 dargestellt. Der Spieler 10 ist durch den abgebildeten Fuß dargestellt. Der Sportstrumpf 1 ist aus einem elastischen Textil, das im unteren Bereich als Fußabschnitt 11 ausgebildet ist, an den sich im oberen Bereich ein Beinabschnitt 12 anschließt.

Der gezeigte Sportstrumpf 1 hat am vorderen Teil des Beinabschnitts 12 ein erstes Dämpfungsteil 21, das das Schienbein eines den Sportstrumpf 1 tragenden Spielers 10 abdeckt und so gegen Schläge schützt. Das erste Dämpfungsteil erstreckt sich über mehr als die Hälfte der Länge des Beinabschnitts. Das erste Dämpfungsteil 21 ist im vorliegenden Beispiel mit einer Breite im Bereich von 10 cm bis 20 cm und einer Höhe im Bereich von 20 cm bis 30 cm dargestellt.

Ein zweites Dämpfungsteil 22 ist an der Rückseite des Beinabschnitts 12 angeordnet, um die Wade eines den Sportstrumpf 1 tragenden Spielers 10 abzudecken. Das zweite Dämpfungsteil 22 liegt damit auf der entgegengesetzten Seite des Beinabschnitts 12 in Bezug auf das erste Dämpfungsteil 21. In der abgebildeten Variante ist das zweite Dämpfungsteil 22 mit einer Breite im Bereich von 5 cm bis 15 cm und einer Höhe im Bereich von 20 cm bis 30 cm ausgeführt.

Ein drittes Dämpfungsteil 23 ist am Übergang zwischen Beinabschnitt 12 und Fußabschnitt 11 angeordnet, so dass dieses die beiden Seiten des Knöchels eines den Sportstrumpf 1 tragenden Spielers 10 abdeckt.

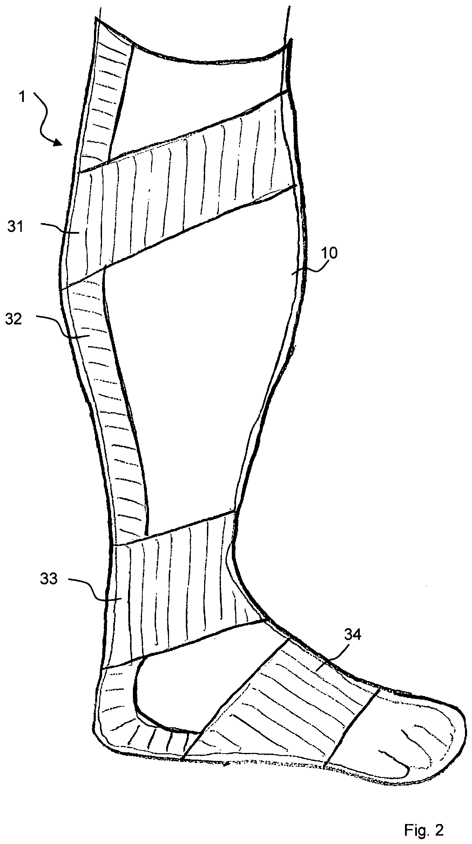

In **Fig. 2** ist die Innenseite des Sportstrumpfs aus Fig. 1 dargestellt, um den Verlauf von Stützbändern zu illustrieren. Alle dargestellten hochelastischen Stützbänder 31, 32, 33, 34 weisen eine im Vergleich zum elastischen Textil im Bereich des Fußabschnitts 11 und des Beinabschnitts 12 höhere Elastizität auf. Die elastischen Stützbänder 31, 32, 33, 34 sind getrennt vom Sportstrumpf aus Fig. 1 dargestellt, jedoch einstückig mit dem Sportstrumpf 1 ausgebildet.

Die hochelastischen Stützbänder sind derart ausgebildet, dass die Rückstellkraft im Vergleich zu dem elastischen Textil um mehr als 30 % bis 60 % größer ist.

Die Ausgestaltung umfasst vier Stützbänder, nämlich ein Wadenstützband 31, ein Knöchelstützband 33, ein Fersenstützband 32 und ein Fußrückenstützband 34.

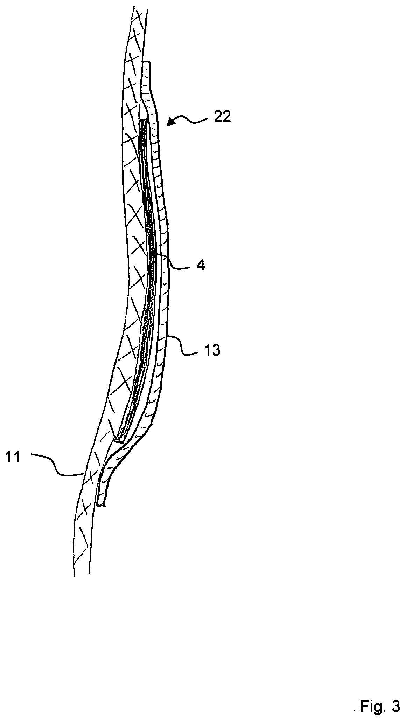

**Fig. 3** zeigt den Aufbau eines Dämpfungsteils für einen Sportsstrumpf aus Fig. 1 im Detail. Eine Kunststoffschale 4 ist zwischen dem elastischen Textil 11 und einem weiteren elastischen Textilabschnitt 13 angeordnet, wobei es bevorzugt ist, dass die Kunststoffschale 4 auf einer Seite eine Polsterschicht (nicht dargestellt) umfasst.

## Patentansprüche

1. Sportstrumpf (1) zum Schutz vor Verletzungen eines Spielers (10), welcher Sportstrumpf (1) ein elastisches Textil (11, 12) mit einem Fußabschnitt (11) und einem Beinabschnitt (12) umfasst, und wobei an dem Sportstrumpf (1)
- mindestens ein erstes Dämpfungsteil (21), das am Beinabschnitt (12) derart angeordnet ist, um das Schienbein eines den Sportstrumpf (1) tragenden Spielers (10) abzudecken,
- mindestens ein zweites Dämpfungsteil (22), das am Beinabschnitt (12) derart angeordnet ist, um die Wade eines den Sportstrumpf (1) tragenden Spielers (10) abzudecken, und
- mindestens ein drittes Dämpfungsteil (23), das am Übergang zwischen Beinabschnitt (12) und Fußabschnitt (11) derart angeordnet ist, um einen Knöchel eines den Sportstrumpf (1) tragenden Spielers (10) abzudecken.

2. Sportstrumpf (1) nach Anspruch 1, wobei das erste Dämpfungsteil (21), das zweite Dämpfungsteil (22) und das dritte Dämpfungsteil (23) eine Kunststoffschale (4) umfasst, und wobei die Kunststoffschale (4) zwischen dem elastischen Textil (11, 12) und einem weiteren elastischen Textilabschnitt (13) angeordnet ist.

3. Sportstrumpf (1) nach Anspruch 2, wobei die Kunststoffschale (4) auf einer Seite eine Polsterschicht, insbesondere eine Schaumstoffschicht, umfasst.

4. Sportstrumpf (1) nach einem der vorangehenden Ansprüche, wobei das elastische Textil (11, 12) zumindest abschnittsweise aus einem transparenten Material ist.

5. Sportstrumpf (1) nach einem der vorangehenden Ansprüche, wobei das dritte Dämpfungsteil (23) einen ersten Dämpfungsteilabschnitt (231), der am Übergang zwischen Beinabschnitt (12) und Fußabschnitt (11) derart angeordnet ist, um eine erste Knöchelseite eines den Sportstrumpf (1) tragenden Spielers (10) abzudecken, und einen zweiten Dämpfungsteilabschnitt (231), der am Übergang zwischen Beinabschnitt (12) und Fußabschnitt (11) derart angeordnet ist, um eine zweite Knöchelseite eines den Sportstrumpf (1) tragenden Spielers (10) abzudecken, umfasst.

6. Sportstrumpf (1) nach einem der vorangehenden Ansprüche, wobei das dritte Dämpfungsteil (23) Befestigungsöffnungen (232) zum Verschnüren mit einem Schuh des den Sportstrumpf (1) tragenden Spielers (10) umfasst.

7. Sportstrumpf (1) nach einem der vorangehenden Ansprüche, wobei das erste Dämpfungsteil (21) eine Breite im Bereich von 10 cm bis 20 cm und eine Höhe im Bereich von 20 cm bis 30 cm hat.

8. Sportstrumpf (1) nach einem der vorangehenden Ansprüche, wobei das zweite Dämpfungsteil (22) eine Breite im Bereich von 5 cm bis 15 cm und eine Höhe im Bereich von 20 cm bis 30 cm hat.

9. Sportstrumpf (1) nach einem der vorangehenden Ansprüche, ferner umfassend hochelastische Stützbänder (31, 32, 33, 34), die eine im Vergleich zum elastischen Textil (11, 12) höhere Elastizität aufweisen, und wobei die elastischen Stützbänder (31, 32, 33, 34) einstückig mit dem Sportstrumpf (1) als Abschnitte des elastischen Textils (11, 12) mit höherer Elastizität oder als separate, am elastischen Textil (11, 12) angeordnete Elemente, mit zum elastischen Textil höherer Elastizität, ausgebildet sind.

10. Sportstrumpf (1) nach Anspruch 9, wobei die Elastizität der hochelastischen Stützbänder derart gewählt ist, dass die Rückstellkraft im Vergleich zu dem elastischen Textil um mehr als 10 % größer ist.

11. Sportstrumpf (1) nach Anspruch 9, wobei die Stützbänder (31, 32, 33, 34) ein Wadenstützband (31), ein Knöchelstützband (33), ein Fersenstützband (32) und ein Fußrückenstützband (34) umfasst.

12. Sportstrumpf (1) nach einem der vorangehenden Ansprüche, wobei das elastische Textil (11, 12) zumindest eines der folgenden Materialien aus der Gruppe Nylon, Kevlar, Glasfasern, Dyneema, Polyester, Polyurethan, Thermoplastisches Polyurethan umfasst.

13. Sportstrumpf (1) nach einem der vorangehenden Ansprüche, wobei das elastische Textil (11, 12) mit einem Kunststoffharz getränkt ist, das derart ausgebildet ist, um bei Kontakt mit Wasser zu erhärten.

14. Sportstrumpf (1) nach Anspruch 2, wobei die Kunststoffschale (4) und das elastischen Textil (11, 12) einstückig ausgebildet ist.
